# EUROPEAN PATENT APPLICATION

(11) **EP 2 106 802 A1**
(43) Date of publication of application: **07.10.2009**
(21) Application number: 08153968.6
(22) Date of filing: 02.04.2008
(51) Int. Cl.: A61K 39/00, C07K 7/08, C07K 7/04, C07K 14/47, A61P 25/28

(54) **Modified peptides as synthetic vaccines in amyloid-associated disease**

(71) Applicant: SIGMA-TAU Industrie Farmaceutiche Riunite S.p.A., 00144 Rome (IT); TECNOGEN S.P.A., 81013 Piana di Monte Verna (IT)
(72) Inventor: Piovesan, Paola, 00177 Rome (IT); Ghirardi, Orlando, 00128 Rome (IT); Verdoliva, Antonio, 80143 Naples (IT); Rossi, Maria, 81100 Caserta (CE) (IT); Colombo, Maurizio, San Giorgio a Cremano (NA) (IT)

(57) **Abstract**

The present invention relates to new immunogenic peptide derivatives, processes for their preparation, and to pharmaceutical compositions containing the same for the prevention and/or treatment of degenerative diseases, such as Alzheimer's disease.

SEQUENCE LISTING
<110>  TECNOGEN S.P.A
       SIGMA-TAU Industrie Riunite S.p.A.
<120>  Modified peptides as synthetic vaccines in amyloid-
associated
       diseases
<130>  553
<160>  11
<170>  PatentIn version 3.3

<210>  1
<211>  42
<212>  PRT
<213>  Artificial
<220>
<223>  Synthetical
<400>  1

<210>  2
<211>  16
<212>  PRT
<213>  Artificial
<220>
<223>  Synthetical
<400>  2
Asp Ala Glu Phe Arg His Asp Ser Gly Tyr Glu Val His His Gln Lys
1               5                   10                  15

<210>  3
<211>  16
<212>  PRT
<213>  Artificial

<220>
<223>  Synthetical
<400>  3
Gly Ala Gly Phe Gly His Gly Ser Gly Tyr Gly Val Gly His Gly Lys
1               5                   10                  15

<210>  4
<211>  16
<212>  PRT
<213>  Artificial
<220>
<223>  Synthetical
<400>  4

Asp Gly Glu Gly Arg Gly Asp Gly Gly Gly Glu Gly His Gly Gln Gly
1               5                   10                  15

<210>  5
<211>  16
<212>  PRT
<213>  Artificial
<220>
<223>  synthetical
<400>  5
His His Gln Lys Leu Val Phe Phe Ala Glu Asp Val Gly Ser Asn Lys
1               5                   10                  15

<210>  6
<211>  16
<212>  PRT
<213>  Artificial
<220>
<223>  Synthetical
<400>  6
Gly His Gly Lys Gly Val Gly Phe Gly Glu Gly Val Gly Ser Gly Lys
1               5                   10                  15

<210>  7
<211>  16
<212>  PRT
<213>  Artificial
<220>
<223>  Synthetical
<400>  7
His Gly Gln Gly Leu Gly Phe Gly Ala Gly Asp Gly Gly Gly Asn Gly
1               5                   10                  15

<210>  8
<211>  18
<212>  PRT
<213>  Artificial
<220>
<223>  Synthetical
<400>  8
Gly Ser Asn Lys Gly Ala Ile Ile Gly Leu Met Val Gly Gly Val Val
1               5                   10                  15
Ile Ala

<210>  9
<211>  18
<212>  PRT
<213>  Artificial
<220>
<223>  Synthetical
<400>  9
Gly Ser Gly Lys Gly Ala Gly Ile Gly Leu Gly Val Gly Gly Gly Val
1               5                   10                  15
Gly Ala

<210>  10

<211>  18
<212>  PRT
<213>  Artificial
<220>
<223>  Synthetical
<400>  10
Gly Gly Asn Gly Gly Gly Ile Gly Gly Gly Met Gly Gly Gly Val Gly
1               5                   10                  15
Ile Gly


<210>  11
<211>  7
<212>  PRT
<213>  Artificial
<220>
<223>  Synthetical
<400>  11
Asp Ala Glu Phe Arg His Asp
1               5

SEQUENCE LISTING

<110>  TECNOGEN S.P.A
       SIGMA-TAU Industrie Riunite S.p.A.

<120>  Modified peptides as synthetic vaccines in amyloid-associated
       diseases

<130>  553

<160>  11

<170>  PatentIn version 3.3

<210>  1
<211>  42
<212>  PRT
<213>  Artificial

<220>
<223>  Synthetical

<400>  1

<210>  2
<211>  16
<212>  PRT
<213>  Artificial

<220>
<223>  Synthetical

<400>  2

Asp Ala Glu Phe Arg His Asp Ser Gly Tyr Glu Val His His Gln Lys
1               5                   10                  15

<210>  3
<211>  16
<212>  PRT
<213>  Artificial

<220>
<223>  Synthetical

<400>  3

Gly Ala Gly Phe Gly His Gly Ser Gly Tyr Gly Val Gly His Gly Lys
1               5                   10                  15

<210>  4
<211>  16
<212>  PRT
<213>  Artificial

<220>
<223>  Synthetical

<400>  4

Asp Gly Glu Gly Arg Gly Asp Gly Gly Gly Glu Gly His Gly Gln Gly
1               5                   10                  15


<210>  5
<211>  16
<212>  PRT
<213>  Artificial

<220>
<223>  synthetical

<400>  5

His His Gln Lys Leu Val Phe Phe Ala Glu Asp Val Gly Ser Asn Lys
1               5                   10                  15


<210>  6
<211>  16
<212>  PRT
<213>  Artificial

<220>
<223>  Synthetical

<400>  6

Gly His Gly Lys Gly Val Gly Phe Gly Glu Gly Val Gly Ser Gly Lys
1               5                   10                  15


<210>  7
<211>  16
<212>  PRT
<213>  Artificial

<220>
<223>  Synthetical

<400>  7

His Gly Gln Gly Leu Gly Phe Gly Ala Gly Asp Gly Gly Gly Asn Gly
1               5                   10                  15


<210>  8
<211>  18
<212>  PRT
<213>  Artificial

<220>
<223>  Synthetical

<400>  8

Gly Ser Asn Lys Gly Ala Ile Ile Gly Leu Met Val Gly Gly Val Val
1               5                   10                  15

Ile Ala

<210>  9
<211>  18
<212>  PRT
<213>  Artificial

<220>
<223>  Synthetical

<400>  9

Gly Ser Gly Lys Gly Ala Gly Ile Gly Leu Gly Val Gly Gly Gly Val
1               5                   10                  15

Gly Ala

<210>  10
<211>  18
<212>  PRT
<213>  Artificial

<220>
<223>  Synthetical

<400>  10

Gly Gly Asn Gly Gly Gly Ile Gly Gly Gly Met Gly Gly Gly Val Gly
1               5                   10                  15

Ile Gly

<210>  11
<211>  7
<212>  PRT
<213>  Artificial

<220>
<223>  Synthetical

<400>  11

Asp Ala Glu Phe Arg His Asp
1               5

## Description

### FIELD OF THE INVENTION

The present invention relates to new immunogenic peptide derivatives, processes for their preparation, and to pharmaceutical compositions containing the same for the prevention and/or treatment of amyloid-associated diseases or conditions, such as for example Alzheimer's disease, Lewy body dementia, inclusion body myositis and cerebral amyloid angiopathy.

### BACKGROUND OF THE INVENTION

Alzheimer disease (AD) is characterized by a progressive loss to cognitive ability. The decline gradually leads to even more serious incapacity to conduct a normal life (loss of memory, incoherent speech, difficulties in carrying common daily tasks, impaired judgement, personality changes) leading to eventual death of the patient.

This disorder is the most common form of dementia in the elderly, affecting 18 million people worldwide (Ritchie K.; Lancet, 2002, 360, 1759) and projected to affect 22 million people by 2025 (Younkin S.G., Nature Medicine, 2001, 7, 18).

Alzheimer's disease not only causes enormous suffering to the persons affected by the conditions and emotional and financial obligation to their caregivers but also to the various governments of residence of the patients.

Alzheimer is characterized in part by extensive deposition of neurofibrillary tangles and senile plaques made of neurotoxic β amyloid peptide (Aβ) in the brain. Aβ is an internal fragment of 39-43 amino acid residues of a precursor protein called amyloid precursor protein (APP).

For many years a lot of efforts have been devoted to the development of selective inhibitors of amyloid formation to reduce the extent of its deposition. Few representative strategies are exemplified underneath.

Few small molecules aiming at tackling Alzheimer's disease through various biological targets have been studied: blocking A β production by directly or indirectly inhibiting β- or γ-secretase activity (Sparey T., Bioorg. Med. Chem. Lett., 2008, 18, 375; Moore, C.L., et al., Exp. Opin. Therap. Patents, 2002, 2, 135), preventing A β aggregation (Porat Y., Chem. Biol. Drug Des., 2006, 67, 27); suppressing the inflammatory response caused by A β deposition (Strauss K.I., Brain Behav. Immun., 2008, 22, 3, 285) and reducing Aβ-mediated neurotoxicity (Rivière C., Bioorg. Med. Chem. Lett., 2008, 18, 828). Unfortunately, none of them so far led to valid therapies.

Over the past few years, new ways of fighting Alzheimer's disease have emerged based on stimulating the immunitary defence system of the patients. Various research groups have reported on the benefits of immunization of different lines of APP transgenic mice with Aβ or derivatives thereof. There are basically two approaches, namely passive and active immunization.

### Passive immunization

It consists at administering manufactured antibodies able to recognize specific epitopes of the Aβ peptide. An epitope typically contains at least 3 amino acids, and more typically at least 8 to 10 amino acids.

WO9960024 describes anti-Aβ antibodies that act through binding pre-formed amyloid deposits (*i*.*e*., plaques) resulting in clearance of localized plaques in the microglia.

WO2001062801 reports murine or humanized monoclonal antibodies (Mab266) that sequesters Aβ[13-28] fragment in APP transgenic mice, interrupting the decline usually observed with the progression of the disease.

A phase II clinical trial involving a monoclonal humanized mAb against β amyloid (ABB-001, Elan Pharmaceuticals Inc/Wyeth Pharmaceuticals) started in April 2005 and is expected to be concluded in April 2008. Passive administration of this mAb, raised against a defined N terminus epitope of Aβ, reduced plaque burden and neuritic pathology to the same degree as active immunization via an Fc-mediated mechanism (Bard F., et al., P. N. A. S., 2003, 4, 2023).

### Active immunization

The first βA active vaccination with aggregated Aβ[1-42] (AN1792; Pharmaceuticals Inc/Wyeth Pharmaceuticals) in a proof-of-concept using APP transgenic mice showed improvement in amyloid pathology (Schenk D.B., et al., Nature 1999, Jul 8, 400, 173). Such immunization also demonstrated delayed cognitive deficits and improved behavioural performance on memory tasks (Janus C., Nature, 2000, 408, 979; Morgan D., Nature, 2000, 408, 982). On the basis of these promising results, clinical trials were performed in United States and Europe in 2001 and 2002. Unfortunately, they were suspended because about 6% of patients of the phase II studies who received the vaccine developed meningoencephalitis, probably mediated by T cells reactive to amyloid peptide (Morgan D., et al., Neurobiol. Aging, 2004, 25, 5, 617). Nevertheless, although the trial was halted, patients who developed antibodies anti-amyloid plaques showed significantly milder decline of cognitive functions than those who did not (Hock C., et al., Neuron, 2003, May 22; 38, 4, 517). In addition, autopsy cases of vaccinated patients underlined the disappearance of senile plaques, thus suggesting that, avoiding potentially harmful proinflammatory processes, vaccination with βA peptides could still be a viable option for the treatment of AD (Nicoll J.A., et al., Nat Med., 2003, Apr 9, 4, 448).

Over the last years, to generate anti-Aβ antibodies that selectively target and remove specific Aβ species without evoking autoimmunity, new and innovative experimental immunization strategies, including selective βA epitope targeting, antibody and adjuvant modifications, as well as alternative routes and mechanisms of vaccine delivery, have been investigated (Hawkes C.A., et al., Expert Rev. Neurother. 2007, Nov 7, 11, 15356). Especially, given the side effects observed with Aβ[1-42] vaccination, the most promising approach in AD immunotherapy is represented by Aβ derivatives with less intrinsic neurotoxicity and reduced overall T-cell responses (Sigurdsson E.M., et al., Am. J. Pathol., 2001, 159, 439; Sigurdsson E.M., et al., 2004, J. Neuroscience, July14,.24, 28, 6277).

WO/2007/068411 describes active immunization with peptidic fragments containing 13 to 15 amino acid residues obtained from the N-terminal fragment [1-20] of the Aβ peptide. Such 13 to 15 amino acid residue fragment can be obtained by deletion of 1 to 3 amino acid residues from the starting Aβ[1-20] peptide fragment. Some analogous fragments have also been obtained by replacement of some residues by chemically similar amino acids. Such fragments were shown to enable the raise of the level of the anti-Aβ antibodies recognizing Aβ[1-42] peptide.

WO/2002/96350 describes active immunization with peptidic fragments containing 10 to 28 amino acids wherein each fragment further comprises the EFRH motif of the Aβ[1-42] peptide. However a further shortening of the short Aβ[1-28] peptide resulted in a lower immunogenicity. It is also noteworthy that no mention of potential inflammatory side-effects is made.

US6743427 discloses active immunization with, among other, Aβ[13-28] peptide. The latter was classified as a less preferred fragment due to its lack of activity.

Wisniewski et al. (WO/2004/087733) identified the peptide Aβ[12-28] as being a promising fragment for active immunization but only after some amino acid residues replacement (i.e.: Val₁₈ with Pro₁₈).

WO/20060/69718 recently reported cyclic Aβ-derived peptides sharing significant sequence homology with Aβ and which constrained conformation allows overcoming the counterproductive flexibility and low metabolic stability of linear peptides. Virosomes and/or liposomes were used as antigen delivery vehicles to increase the relatively low immunogenicity of the amyloid peptide antigen.

It has been reported lately that, ACC-001 (Elan Pharmaceuticals Inc/Wyeth Pharmaceuticals), an Aβ[1-7] peptide fragment is currently involved in a phase II clinical trial.

However, to date no effective cures or treatments being available combined with an aging population, makes Alzheimer's disease the health care crisis of the 21^{st} century. Therefore there is a high need to have at disposition new ways of treating Alzheimer's disease, which do not present the drawbacks identified so far such as inflammation.

The growing interest in potential application of pseudopeptides and peptidomimetics in immunology, including synthetic vaccines, has lead to a deeper investigation on the stereochemical requirements, in terms of chirality and backbone modifications, for peptide antigenicity and immunogenicity (Guichard G., Pept. Res., 1994, 7, 308).

Recent works on topological related peptides showed, in different systems, a generalized cross-antigenicity between the normal peptides and their topological variants obtained by inversion of the side chain chirality (*inverso* derivative), inversion of backbone polarity *(retro* derivative) or by both modifications (*retro-inverso* derivative).

The structural basis of this cross-antigenic recognition has been hypothesized to reside into the molecular surfaces formed by the side chains of alternating residues, which are shared by all topological variants (Verdoliva A., J. Biol. Chem. 1995, 270, 51, 30422). The hypothesis of an antibody/antigen interaction related to "trans-surfaces" of recognition, has been confirmed by experiments with sequence-simplified variants of immunogenic peptides, one lacking of the side chains in the sequence odd position (-G form), and the other in even position (+G form) (Rossi M., et al., Molecular Immunology, 2002, 39, 4439). This modification (glycine substitution), by reducing the number of chiral centers and increasing chain flexibility, should allow the simplified structure to adopt a configuration very similar for all topological related forms, thus overcoming the structural differences introduced by inversion of chirality and backbone direction.

It has now been found a new class of Aβ derivatives as a safer second-generation AD vaccine.

### DESCRIPTION OF THE INVENTION

To avoid toxicity and autoimmune response of the immunogen in the amyloid system, we have designed a new class of Aβ derivatives through segmentation of original Aβ[1-42] and application of the glycine substitution modification technology, consisting in sequence modification of Aβ[1-42] fragments by introduction of glycines in alternate positions, at even positions (+G) or odd positions (-G), of the sequence. Aβ[1-16], Aβ[13-28] and Aβ[25-42] fragments of the Aβ[1-42] sequence were selected in such a way to retain the major immunogenic sites of Aβ[1-42] peptide, corresponding to residues [1-11], [4-7] and [22-28] based on an antigenic index (Jameson B.A., et al., Comput. Appl. Biosci., 1988, 4, 181). These three sequences and corresponding glycinated forms (that means +G and -G) were then synthesized and characterized for their fibrillogenic, aggregating, immunogenic and antigenic properties to be compared to the Aβ[1-42] peptide.

All peptides maintained their immunogenic activity, and raised antibodies were all able to cross-recognize Aβ[1-42] and Aβ[1-40] synthetic peptides. Polyclonal antibodies produced against the modified variants were able to recognize the parent peptide, while antibodies anti (+G) modified peptide were not able to cross-react with the corresponding (-G) modified molecule and vice versa. These results showed that by introducing glycine residues, it was possible to obtain modified peptides maintaining the main immunogenic properties of the parent peptides.

In addition, all Aβ derivatives showed reduced fibrillogenic and aggregating properties. In particular, both Aβ[1-16] and Aβ[13-28] forms, and corresponding (+G) and (-G) modified structures, showed a reduced ability to adopt a β-sheet conformation and therefore a much lower risk of toxicity in humans. *In vitro* studies on peripheral blood mononuclear cells (PBMCs) from healthy donors showed that Aβ[1-16]+G and Aβ[13-28]+G modified peptide did induce IFN-γ production very mildly or not at all respectively.

This finding shows that the modified Aβ[1-16]+G and Aβ[13-28]+G derivatives represent good candidates for safer vaccine therapy to reduce amyloid burden in Alzheimer's disease instead of using toxic Aβ[1-42].

Therefore the main embodiment of the present invention is that of a vaccine useful to prevent or to treat an amyloid-related disease or condition.

Preferably a further embodiment of the present invention is that of a vaccine, containing a modified amyloid peptide fragment aiming at interacting with amyloid proteins to prevent fibrillogenesis.

In a more preferred embodiment of the present invention, the modified amyloid peptide consists of a fragment of the amyloid peptide Aβ within the [1-28] region ,wherein each amino acid in even position is replaced by the amino acid glycine.

In an even more preferred embodiment of the present invention, said vaccine contains a modified amyloid peptide fragment consisting of 7 to 18 amino acid residues, more preferably of 16 or 17 amino acid residues.

In another even more preferred embodiment of the present invention, the modified amyloid peptide is obtained by replacement of the amino acid residues in the even positions by glycine residues.

In another still even more preferred embodiment of the present invention, said peptide consists of the fragment Aβ[1-16]+G or the amino acid sequence of SEQ ID NO: 4.

In a still even more preferred embodiment of the present invention, said peptide consists of the fragment Aβ[13-28]+G or the amino acid sequence of SEQ ID NO: 7.

The peptides were synthesized following standard peptidic coupling conditions involved in solid phase synthesis using the N-Fmoc protection strategy.

The peptides of this invention may be easily prepared according to conventional techniques of the peptide chemistry that comprise suitable protection of amino acids, coupling of protected amino acids and removal of protecting groups.

Preferably, they are prepared according to the solid phase synthesis technique, which comprises the following steps:
➢ coupling, using suitable reactants, of the first amino acid from the C-terminus protected by suitable groups both in the amino group and, when required, in the side chain, on a suitable support for the solid phase synthesis,
➢ removal of the protecting groups at the amino moiety with suitable reactants,
➢ coupling of the following amino acid from the C-terminus, protected at the amino moiety and, when required, in the side chain,
➢ removal of the protecting group at the amino moiety and repetition of the coupling and deprotection cycles until all the amino acids constituting the peptide sequence have been assembled,
➢ removal of all remaining side chain protecting groups from the assembled peptide and cleavage from the resin.

These techniques are widely described in the literature and well known to the person skilled in the art (Atherton E., et al., 1989, Solid Phase Synthesis: A pratical approach, IRL Press, Oxford, UK; Bodansky M., et al., 1994, The Practice of Peptide Synthesis, 2nd Ed. Springer Verlag, Berlin).

Amyloid deposits are also a key feature in a series of diseases or conditions comprising Lewy body dementia (Mann D.M.A., et al, Neuropathol. Appl. Neurobiol., 1998, 24, 3, 187), inclusion body myositis (Askanas V., et al., Curr. Opin. Rheumatol., 2003, 15, 6,737) and cerebral amyloid angiopathy (Smith E.E., et al., Arch. Neurol., 2006, 63, 148).

Therefore one of the embodiments of the invention is the use of the claimed peptide for the treatment and/or prevention of one of the above-mentioned diseases or conditions.

The present invention also provides pharmaceutical compositions for the prophylaxis and/or treatment of the above-mentioned diseases, comprising the immunogenic peptide of the invention as active ingredient.

Another object of the present invention is a pharmaceutical composition comprising an antigenic peptide of the invention aiming at stimulating the production of antibodies directed to cross-react with Aβ[1-40] and/or Aβ[1-42].

Still, another object of the present invention is a method of producing a therapeutic vaccine comprising an active ingredient consisting of the peptide of the invention conjugated or not to a carrier.

Pharmaceutical compositions comprising the immunogenic peptide of the present invention include all compositions wherein said immunogenic peptide is contained in therapeutically effective amount, that is, an amount effective to achieve the medically desirable result in the treated animal.

The pharmaceutical compositions may contain suitable pharmaceutical acceptable carriers, biologically compatible vehicles suitable for administration to an animal (for example, physiological saline) and eventually comprising auxiliaries (like excipients, stabilizers or diluents) that facilitate the processing of the active immunogenic peptides into preparations, which can be used pharmaceutically. A non-exhaustive list of pharmaceutical acceptable carriers comprises Keyhole Limpet Hemocyanin immunoglobulin molecules, thyroxinebinding globulin, tetanus toxoid and bovine serum albumin (BSA).

The pharmaceutical compositions may be formulated in any acceptable way to meet the needs of the mode of administration. The use of biomaterials and other polymers for drug delivery, as well the different techniques and models to validate a specific mode of administration, are disclosed in literature. Modifications of the immunogenic peptide of the invention to improve penetration of the blood-brain barrier would also be useful.

Vaccine: As used herein, the term "vaccine" refers to a formulation that contains the composition of the present invention in a form that is capable to be administered to an animal and to trigger an immune response.

Any accepted mode of administration can be used and determined by those skilled in the art. For example, administration may be by various parenteral routes such as subcutaneous, intravenous, intradermal, intramuscular, intraperitoneal, intranasal, transdermal, oral, or buccal routes. Parenteral administration can be by bolus injection or by gradual perfusion over time. Preparations for parenteral administration include sterile aqueous or non-aqueous solutions, suspensions, and emulsions, which may contain auxiliary agents or excipients known in the art, and can be prepared according to routine methods. In addition, suspension of the active immunogenic peptide as appropriate oily injection suspensions may be administered. Suitable lipophilic solvents or vehicles include fatty oils, for example, sesame oil, or synthetic fatty acid esters, for example, sesame oil, or synthetic fatty acid esters, for example, ethyloleate or triglycerides.

Aqueous injection suspensions that may contain substances increasing the viscosity of the suspension include, for example, sodium carboxymethyl cellulose, sorbitol, and/or dextran. Optionally, the suspension may also contain stabilizers. Pharmaceutical compositions include suitable solutions for administration by injection, and contain from about 0.01 to 99 percent, preferably from about 20 to 75 percent of active immunogenic peptide together with the excipient. Compositions that can be administered rectally include suppositories.

It is understood that the dosage administered will be dependent upon the age, sex, health, and weight of the recipient, kind of concurrent treatment, if any, frequency of treatment, and the nature of the effect desired. The dosage will be tailored to the individual subject, as is understood and determinable by one of skill in the art. The total dose required for each treatment may be administered by multiple doses or in a single dose. The pharmaceutical composition of the present invention may be administered alone or in conjunction with other therapeutics directed to the condition, or directed to other symptoms of the condition. Usually a daily dosage of active ingredient is comprised between 0.01 to 100 milligrams per kilogram of body weight. As well known in the medical arts, dosages for any one patient depend upon many factors, including the patient's size, body surface area, age, the particular immunogenic peptide to be administered, sex, time and route of administration, general health, and other drugs being administered concurrently. According to the present invention the term "active ingredient" refers to an antigenic peptide and/or antigenic peptide conjugated to a carrier contained in the vaccine.

The vaccines of the present invention may be administered to the patient intravenously in a pharmaceutical acceptable carrier such as physiological saline.

Standard methods for intracellular delivery of peptides can be used, e.g. delivery via liposomes. Such methods are well known to those of ordinary skill in the art. The formulations of this invention are useful for parenteral administration, such as intravenous, subcutaneous, intramuscular, and intraperitoneal.

All references cited herein are entirely incorporated by reference herein, including all data, tables, figures, and text presented in the cited references. Additionally, the entire contents of the references cited within the references cited herein are also entirely incorporated by reference. Reference to known method steps, conventional method steps, known methods or conventional methods is not in any way an admission that any aspect, description or embodiment of the present invention is disclosed, taught or suggested in the relevant art.

Once understood the features of the methods and products disclosed in present application, the necessity and kind of additional steps can be easily deduced by reviewing prior art, as well as the non-limiting following figures and examples describing the basic details and some applications of the invention.

### DESCRIPTION OF THE DRAWINGS

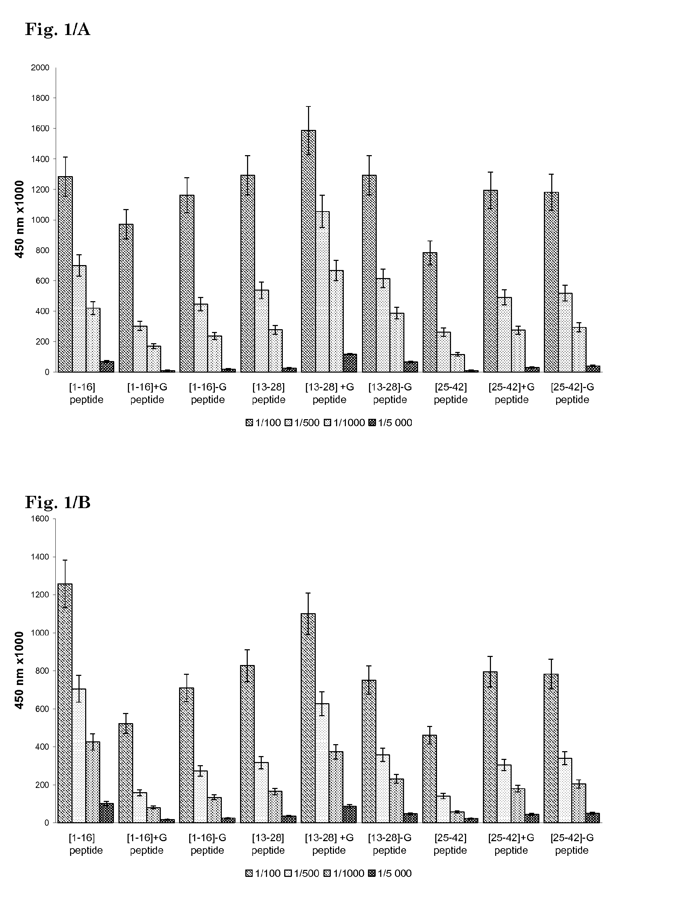

Figure 1 Immunization with modified peptides Aβ[1-16], (+G) and (-G), Aβ[13-28], (+G) and (-G), Aβ[25-42], (+G) of Balb/c mice elicited the production of IgG able to cross-bind Aβ[1-42] (Panel A) and Aβ[1-40] (Panel B) peptides coated on ELISA microplates. Plasma samples were diluted in the range 1:100-1:5000.

Animals received peptides conjugated to KLH and adjuvant while Aβ[1-42] and Aβ[1-40] conjugated with BSA were used for coating in ELISA plates.

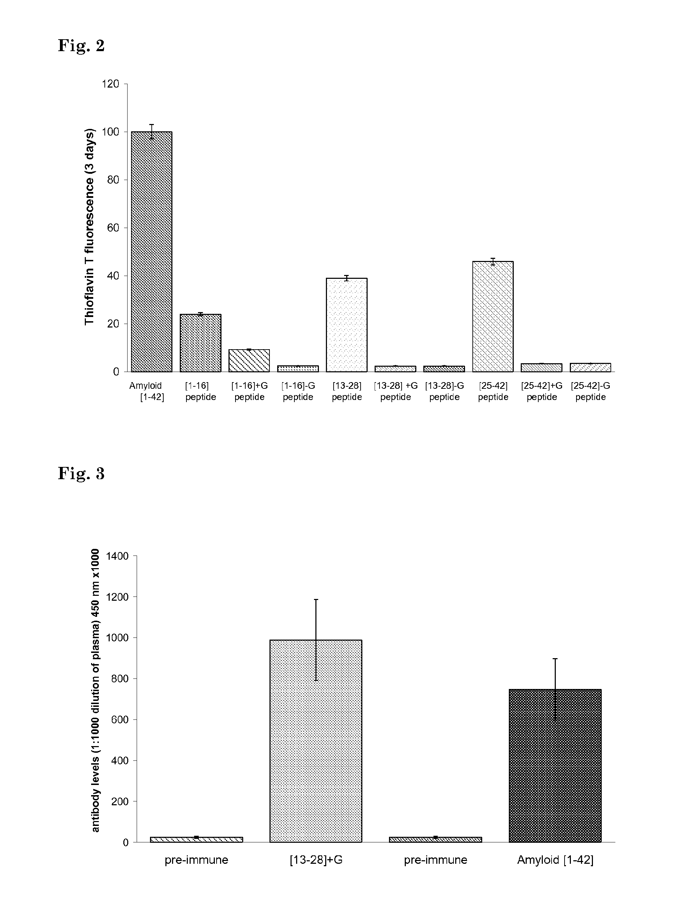

Figure 2. Thioflavin T fluorometric assay. Fibril formation of Aβ[1-42], of peptides Aβ[1-16], Aβ[13-28] and Aβ[25-42] and of modified forms (+G) and (-G) was measured *in vitro* in triplicates (37°C- 3d) by fluorometric assays based on thioflavin T-fluorescence emission.

Figure 3. Immunization with Aβ[13-28]+G of C57BL/6 mice resulted in a good increase in IgG recognizing the antigen and Aβ[1-42] on ELISA plates as detected in plasma samples diluted 1:1000 obtained at the end of the study. Controls are pre-immune plasma samples diluted in the same way and incubated on the same peptide-coated ELISA plates Animals received peptide conjugated to KLH and adjuvant while Aβ[13-28]+G and Aβ[1-42] both being conjugated with BSA were used for coating in ELISA plates.

### EXAMPLES

### Example 1

### Materials and Methods

### Peptides synthesis

The peptides were produced by solid phase synthesis following the Fmoc protection methodology on a fully automated Model ABi 431A (Applied Biosystems) peptide synthesizer, software version 1.2 as described previously (Verdoliva A., J. Biol. Chem. 1995, 270, 51, 30422). After resin cleavage, low molecular weight contaminants were removed by dialysis against 0.1M acetic acid. Peptides were purified by RP-HPLC on a C18 (Phenomenex) column. Peptide identity was confirmed by molecular weight determination by TOF-MALDI mass spectrometry.

### Peptides-carrier conjugates

For mice immunization, linear peptides were covalently coupled to Keyhole Limpet Hemocyanin (KLH) (Sigma N. H7017), while, to allow coating to polystyrene microtiter plates (Falcon, 3912) in ELISA experiments, peptides were conjugated to bovine serum albumin (BSA, Sigma N. A7030). All conjugations were performed using glutaraldehyde (Sigma N. G5882) cross-linking between the carrier and the *N*-terminal part of the corresponding peptides.

Three micrograms of peptides were dissolved in 1 ml of 50 mM, sodium phosphate buffer pH 7.0 (PBS, Sigma-Aldrich) and added to 1 ml of carrier solution (9 µg dissolved in the same buffer). The mixture was incubated with 2 ml of 0.2% glutaraldehyde in 50 mM PBS and after 1 hr of incubation at room temperature (rt), 1 ml of 1 M Gly (Sigma, G8898) in 50 mM of PBS was added. One hour later, the conjugates were extensively dialyzed against 50 mM PBS, pH 7.0.

### Animals

The animals, Balb/c mice (Harlan, Italy) and C57 BL/6 mice (Taconics), were maintained on a 12 hr light/dark cycle and animal care was in accordance with institutional guidelines.

### Anti-peptide antisera

Mouse anti-peptide antisera were prepared using peptide-keyhole limpet hemocyanin (KLH) conjugates. Different mouse strains, Balb/c mice (Harlan Italy) and C57 BL/6 (Taconics) were immunized with peptides dissolved in 0.15 M sodium chloride 0.05 M sodium phosphate buffer, pH 7.0 and emulsified with different adjuvants such as TiterMax gold (Sigma, T2684), Alum (Sigma) and Freund's (Sigma).

Balb/c mice received 30 µg of each peptide dissolved in 50 µl phosphatebuffered saline (PBS) and mixed with 50 µl of adjuvant TiterMax gold. The mice received a first intraplantar injection of the antigens followed by a second injection 4 weeks later.

Alternatively, 30 µg of peptides were dissolved in PBS and mixed with the adjuvant Alum (1 mg) and animals received the immunogens by intraperitoneal injections. In this case, mice received an injection of 200 µl followed by a successive injection (30 µg/ 200 µl) 4 weeks later. Regarding C57 BL/6 mice, the peptides were dissolved in PBS at a concentration of 2 mg/ml and then mixed 1:1 (v/v) with the Freund's adjuvant. Complete Freund's adjuvant was used for the first injection and incomplete Freund's adjuvant for the next injections. The mice received a subcutaneous injection of 100 µl, followed by a second 100 µl injection 2 weeks later and then 4 weeks later of the same volume.

### Enzyme linked immunosorbent assay (ELISA)

Polystyrene microtiter plates were incubated overnight at 4°C or for 2 hr at rt, in a humid covered box, with 50 µl per well of peptides conjugated to BSA at the concentrations of 20-40 µg ml⁻¹ in PBS, pH 7.0. Following four washings with PBS solution, the wells were saturated with 100 µl of PBS containing 3% dried milk for 2 hr at rt, to block the uncoated plastic surface. Plates were then washed again with PBS and filled with samples containing antibodies at varying concentrations, previously diluted with PBS containing 0.5% dried milk (PBS-M). Antibody levels were determined by 1:200 and 1:20000 dilutions of plasma (data not shown). After 1-2 hr of incubation, the plates were washed four times with PBS. For antibody detection, wells were filled with 50 µl of a horseradish peroxidise labelled goat anti-mouse immunoglobulin (IgG) diluted 1:1000 with PBS-M or alternately with 50 µl of a goat antimouse IgG (whole molecule) alkaline phosphate conjugate (Sigma, A3562) diluted 1:1000 with PBS-M and with 50 µl of goat anti-mouse IgM alkaline phosphate conjugate (Sigma, A7784) diluted in the same way. The plates were then left for 1 hr at rt, washed five times with PBS, and then filled with chromogenic substrate solution consisting of 1mg/ml 2,2-azino-bis(3-ethylbenzthiazoline-6-sulfonic acid, Sigma, A1888) in 0.1M sodium citrate phosphate buffer, pH 5.0, containing 5 mM hydrogen peroxide or alternately of p-nitrophenyl phosphate substrate (pNPP, Sigma, N2770).

The absorbance at 405 nm was determined with a Microplate Reader Model 3550 EIA (Bio-Rad).

For cross-reactivity experiments, antibody levels were determined by 1:100-1:5000 dilutions of immune plasma using ELISA in which Aβ[1-42]-BSA and

Aβ[1-40]-BSA were coated onto microtiter wells.

### Thioflavin T assay.

Aliquots of the peptides prepared in 0.1 M Tris, pH 7.4, were incubated for different times (1-3 days), and their fibril formation determination was compared with that of Aβ[1-42]. *In vitro* fibrillogenesis was evaluated by a thioflavin T assay, as described previously (Soto C., et al., Nat. Med., 1998, 4, 822). The specific binding of thioflavin T to amyloid shifts its emission spectrum, producing a fluorescent enhancement proportional to the amount of amyloid formed.

### RP-HPLC analysis.

Aliquots of 1 mg of each peptide, dissolved in 5% DMSO/PBS pH 7.0, were incubated for 8 days at 37° C and their aggregation activity was compared to that of Aβ[1-42]. Samples were centrifuged at 13.000 rpm for 5 min, supernatants were diluted 1:1 with H₂0 /TFA 100/0.1 (v/v) and analyzed by RP-HPLC. Aggregated peptides were treated with formic acid (50 µl), diluted 1:1 with H₂0 /TFA 100/0.1 (v/v) and analyzed by RP-HPLC using RP1 and Jupiter **RP18 columns to analyze Aβ[1-42] and modified peptides respectively.**

### Immunogenic activation in PBM cells from healthy donors

The aim of the experiment was to identify a peptide capable to induce a response to stimulation in follicular T helper lymphocytes, a subpopulation of which, that expressing CXCR5, supports the B lymphocytes in the Aβ-mediated immune response, without eliciting any inflammatory response. To do this, the T-lymphocytes form peripheral blood mononuclear cells (PBMCs) response to Aβ[1-42] and Aβ[1-16], Aβ[1-16]-G, Aβ[1-16]+G, Aβ[13-28], Aβ[13-28]-G, Aβ[13-28]+G, Aβ[25-42], Aβ[25-42]-G, Aβ[25-42]+G was studied with the following experimental procedure:

### Cell preparation and T cell lines

In order to obtain mature dentritic cells, PBMCs from healthy donors were isolated by Ficoll-Hypaque gradient centrifugation (Pharmacia, Uppsala, Sweden). The cells were washed twice with PBS and finally were cultured at 10⁶ cells/ml in RPMI 1640 supplemented with 10% (v/v) heat inactivated human serum (Bio Whittaker; Cambrex, Bioscience, Walkersville, MD Usa), 2mM L-glutamine, 20mM HEPES [*N-*2-hydroxyethylpiperazine-*N'*-2-ethanesulfonic acid], 1mM sodium pyruvate, 20mM non-essential amino acids, 55 µM 2-β-mercaptoethanol, 10 U/ml penicillin and streptomycin (Gibco and Invitrogen).

### In vitro culture of monocyte-derived dendritic cells

In order to obtain a pure culture of human peripheral blood monocytes, cells were purified by plastic adherence at 37°C for 30 minutes, followed by extensive washing with warm PBS to remove non-adherent cells. The monocytes were cultured in six-well plates (0,5 to 1,5 * 10⁶ cells/m) in fresh complete medium supplemented with 1000U/ml of recombinant granulocyte-macrophage colony-stimulating factor (rGM-CSF) and 500U/ml interleukin-4 (IL-4, Pharmigen) for four days. The non-adherent mononuclear cells were washed repeatedly and cultured in 96-well round bottom plates (Nunc, Denmark) (10⁶ cell/ml) in complete culture medium supplemented with interleukin-2 (IL-2; 5U/ml; Roche) for 4 days and used for subsequent studies. Monocytes-derived immature dendritic cells (DCs) were stimulated with the Aβ[1-16], Aβ[1-16]-G, Aβ[1-16]+G, Aβ[13-28], Aβ[13-28]-G, Aβ[13-28]+G, Aβ[25-42], Aβ[25-42]-G, Aβ[25-42]+G and Aβ[1-42] peptides (5ug/ml) for 1 day. For induction of dentritic cells (DC) maturation, the cells were stimulated with 50ng/ml TNFα (Pharmigen), 10ng/ml IL-6, 10ng/ml IL-1β, and 1µg/ml PGE2 for additional 2 days. Finally, they were cultured with autologous T-lymphocytes in 96-well flat bottom plates at a 1:4 ratio. A second stimulation with mature autologous monocyte-derived DC's previously stimulated with the peptides was performed at 10-14 days at the same ratios. Following further 10-14 days, lymphocytes were labelled with CFDA challenged with a third stimulation of monocyte-derived DC in the presence of peptides.

### Flow cytometry

In order to evaluate the expression of a specific marker (CXCR5) in cultured monocytes, the cells, 4 days after the third stimulation, were washed twice and stained with anti-CXCR5, in PBS supplemented with 0.5% FCS and 0.01%NaN₃ at 4°C. After twenty minutes they were washed twice and analysed on FACSCanto flow cytometer (Becton Dickinson). The data were analyzed using FlowJo software (TreeStar, CA, USA).

### Enzyme-linked immunosorbent assay (ELISA)

In order to evaluate the amount of IFN-γ produced by lymphocytes after the final third peptides stimulation the supernatants from cultured cells were harvested at 24 or 48 hr after the third stimulation. Cytokines were quantified by a standard-2-site sandwich enzyme-liked immunosorbent assay (ELISA). Abs for human IFN-γ were purchased from Pharmigen. Enhanced protein-binding ELISA plates (Nunc Maxisorb, Nunc Maxi, Roskilde, Denmark) were used.

Table 1 shows the peptidic sequences of the Aβ[1-42] and of its selected derivatives, including positive and negative glycinated Aβ[1-16], Aβ[13-28], Aβ[25-42] peptides as well as of Aβ[1-7].

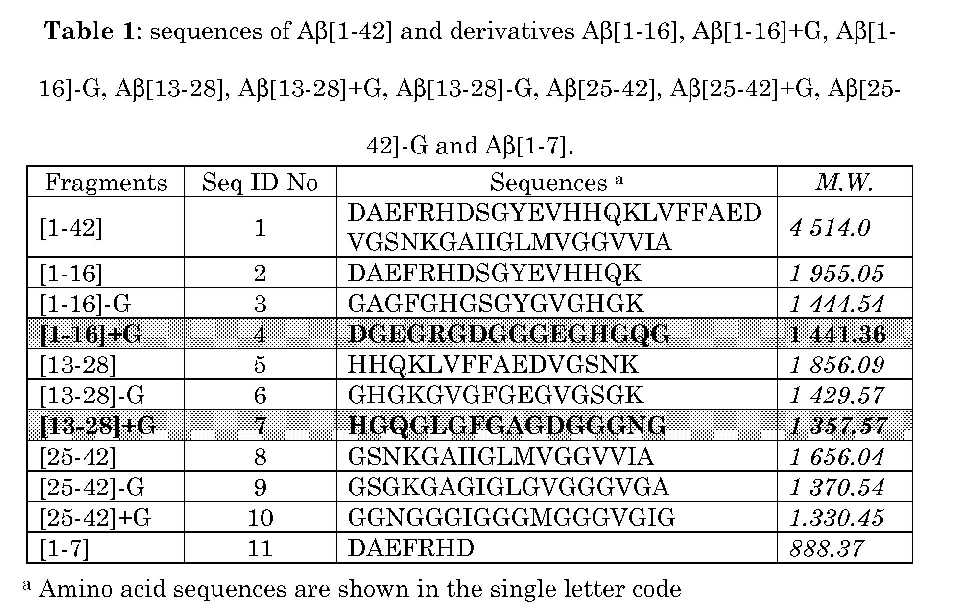

### Results

Table 2. Aggregated formation assay by RP-HPLC. Formation of aggregates of Aβ[1-42] and of peptides Aβ[1-16], Aβ[13-28], Aβ[25-42] and of all modified forms(+G) and (-G) was tested by RP- HPLC (37°C-8 d).

Table 3. The CXCR5 expression measured as % of T lymphocytes expressing CXCR5 in PBMCs of healthy donors vs total lymphocytes (median, 1^{st} and 3^{rd} quartile) has been reported. Friedman Repeated Measures Analysis of Variance on Ranks has been performed. Column 1: number of different donors (Pz); column 2: the percentage of control (CTR) not challenged T lymphocytes expressing CXCR5; columns 3-12: the percentage of T lymphocytes expressing CXCR5 after the stimulation with different peptides.

Table 4. The T lymphocytes activation after peptides stimulations, measured as released IFN-γ (pg/ml), has been reported. Column 1: name of different donors (Pz); column 2: the released IFN-γ (pg/ml) from control (CTR), not challenged, T lymphocytes; columns 3-11: the released IFN-γ (pg/ml) from challenged T lymphocytes. Fisher's exact test has been performed on the number of responding patients (patients whose lymphocytes released IFN-γ) and not responders patients (patients whose lymphocytes did not release IFN-γ). Differences not statistically significative have been reported as "n.s.".

All peptides were able to induce a strong immunogenic response in mice even after the 1^{st} boost, as detected by linear binding ELISA assays performed with the corresponding antigens conjugated to BSA to facilitate their coating on polystyrene microtiter plates and to exclude anti-KLH antibody recognition.

Interestingly, as shown in figure 1, all produced antibodies were able to cross-recognize Aβ[1-42] (panel A) and Aβ[1-40] (panel B) synthetic peptides, in a dose-dependent manner. In these assays, antibody titers were determined by serial dilutions of sera (1:100-1:5000) using an ELISA assay in which the Aβ[1-42] and Aβ[1-40] were coated onto microtiter wells. No differences in antibody production were observed when using various adjuvants or when various mouse strains were involved in the experiments (data not shown).

To evaluate the feasibility of using multimeric peptides (MAP) to improve the immunogenicity and avoid carrier conjugation, Aβ[1-16], Aβ[13-28] and corresponding modified (+G) and (-G) structures were synthesized in tetrameric form and used to immunize Balb/C mice. The immunization procedure was performed as previously described, using both TiterMax gold and Alum as adjuvants. Antibody production was monitored by ELISA assays. Titers were significantly lower in mice immunized with multimeric peptides without KLH-conjugation, thus suggesting that the use of carrier-conjugation was required to elicit stronger immunogenic responses.

We have characterized Aβ derivatives, including +G and -G corresponding modified peptides for their ability to aggregate (table 2) and to adopt β-sheet conformation (figure 2). Aβ[1-16] and Aβ[13-28] modified peptides showed reduced fibrillogenic properties and high solubility in comparison to their parent forms and Aβ[1-42], while all Aβ[25-42] derivatives, containing the cytotoxic Aβ[25-35] region (Pérez M., et al., J. Alzheimer Dis., 2004, 6, 5, 461), showed reduced fibrillogenic activity, but lower solubility with reference to others peptides. These findings suggest that a potentially lower risk of toxic effects in humans could be associated with Aβ[1-16] and Aβ[13-28] modified derivatives.

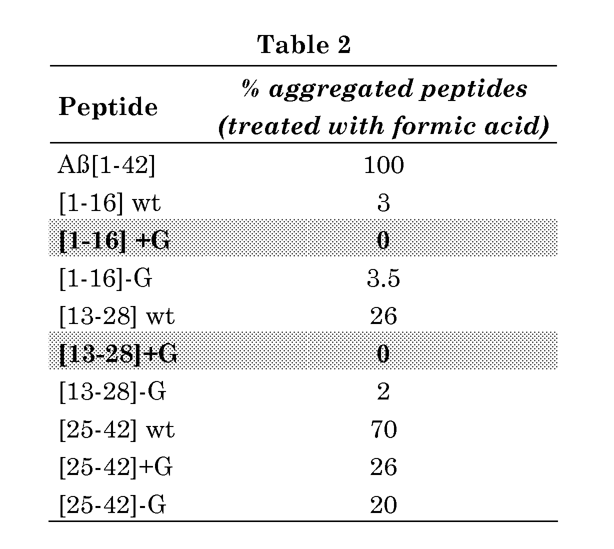

The aseptic meningoencephalitis observed in the first AD vaccination trial (AN-1792, Elan Pharmaceuticals Inc/Wyeth Pharmaceuticals) finds its cause in T cell infiltration in the brain mediated by TGF-β1 overproduction. This mechanism can be enhanced in particular after Aβ[1-42] immunization (Buckwalter M.S., et al., J. Neurosci., 2006, 26, 44, 11437). Consequently, the development of new immune-based AD therapies is critically dependent on the ability to limit toxicity.

In this way, to evaluate the inflammatory properties of described Aβ derivatives, the T-lymphocytes response to Aβ[1-16], Aβ[1-16]-G, Aβ[1-16]+G, Aβ[13-28], Aβ[13-28]-G, Aβ[13-28]+G, Aβ[25-42], Aβ[25-42]-G, Aβ[25-42]+G and Aβ[1-42] peptides stimulations were evaluated in peripheral blood mononuclear cells (PBMCs) from different healthy donors. After the challenge, two different T-lymphocytes subpopulations of responders, corresponding to CXCR5 positive T-lymphocytes and pro-inflammatory T-lymphocytes producing IFN-γ, were identified.

CXCR5, a chemokine transmembrane receptor belonging to the CXC chemokine receptor family plays an essential role in B cell activation (Legler D.F., et al., J. Exp. Med., 1998, 187, 4, 655). The CXCR5 expression was measured as % of T-lymphocytes expressing CXCR5 *versus* total lymphocytes (median, 1^{st} and 3^{rd} quartile) and Friedman Repeated Measures Analysis of Variance on Ranks was performed.

All the examined Aβ[1-16], Aβ[1-16]-G, Aβ[1-16]+G, Aβ[13-28], Aβ[13-28]-G, Aβ[13-28]+G, Aβ[25-42], Aβ[25-42]-G, Aβ[25-42]+G and Aβ[1-42] peptides were able to elicit, in T-lymphocytes, a statistically different expression of CXCR5 with respect to CTR (Table 3, χ²=34.15, p0.001). In addition, there was no statistically different induced CXCR5 expression among the different peptides in T-lymphocytes, thus confirming that all derivatives were able to induce a similar specific immune response.

**Table 3: Percentage of T-lymphocytes expressing CXCR5 in PBMC's of Healthy Donors**

| | **CTR** | **Seq.1** | **Seq.2** | **Seq.3** | **Seq.4** | **Seq.5** | **Seq.6** | **Seq.7** | **Seq.8** | **Seq.9** | **Seq.10** |
|---|---|---|---|---|---|---|---|---|---|---|---|
| **Pz1** | 13.08 | 19.06 | 23.09 | 16.05 | 19.08 | 15.05 | 22.04 | 21.06 | 19.00 | 23.01 | 13.05 |
| **Pz2** | 10.00 | 15.07 | 21.09 | 20.05 | 23.01 | 23.05 | 23.03 | 21.07 | 13.06 | 15.02 | 15.07 |
| **Pz3** | 13.00 | 68.0 | 83.07 | 64.01 | 38.00 | 12.08 | 25.09 | 55.09 | 64.04 | 11.03 | 20.04 |
| **Pz4** | 0,8 | 0.09 | 2.08 | 2.09 | 1.03 | 1.02 | 0 | 0 | 0.08 | 0.08 | 1.02 |
| **Pz5** | 7,5 | 8,6 | 9,9 | 9,5 | 10,2 | 8,5 | 13,4 | 11,6 | 8,2 | 11,3 | 9,2 |
| **Pz6** | 5,2 | 7,5 | 6,9 | 6,9 | 7,1 | 7,4 | 7,3 | 7,6 | 7,3 | 7,5 | 7,4 |
| **Pz7** | 6,8 | 13,3 | 14,8 | 14,7 | 14,1 | 13,5 | 14,6 | 15,6 | 15,8 | 13,4 | 14,1 |
| **Pz8** | 20.01 | 38.02 | 44.00 | 40.06 | 39.08 | 45.01 | 43.03 | 52.02 | 45.00 | 61.07 | 34.02 |
| **Pz9** | 15.01 | 40.07 | 46.05 | 35.03 | 39.09 | 33.00 | 31.03 | 39.09 | 36.07 | 36.01 | 33.07 |
| **Pz10** | 17.00 | 40.02 | 38.01.00 | 36.00 | 44.01 | 30.04 | 29.05 | 38.00 | 36.00 | 34.06 | 33.04 |
| **Median** | 11.05 | 17.7* | 22.9* | 30.6* | 18.5* | 14.5* | 21.7* | 22.9* | 17.4* | 14.9* | 14.3* |
| **1^{st} and 3^{rd} quartine** | 6.8-15.1 | 8.6-40.2 | 9.9-44.0 | 14.1-39.9 | 9.5-36.0 | 8.5-30.4 | 11.3-39.9 | 13.4-29.5 | 8.2-36.7 | 9.2-33.4 | 11.3-34.6 |

| | | | | | | | | | | | |
|---|---|---|---|---|---|---|---|---|---|---|---|
| Friedman Repeated Measures Analysis of Variance on Ranks χ ²=34.15; DF=11; *: P = <0,001 | | | | | | | | | | | |

The T-lymphocytes activation after peptides stimulations was measured as release of IFN-γ, a pro-inflammatory cytokine involved in the innate and acquired immune responses, which promotes the differentiation of naive helper T cells into Th1 cells, activates polymorphonuclear leucocytes (PMN) and cytotoxic T cells and increases the cytotoxicity of NK cells.

Fisher's exact test was performed on the number of responding patients (patients whose lymphocytes released IFN-γ) and not responding patients (patients whose lymphocytes did not release IFN-γ).

Analysis of IFN-γ released by PMB cells stimulated with Aβ[1-42] derivatives is showed in table 4. Data show that Aβ[1-16], Aβ[1-16]-G, Aβ[13-28], Aβ[13-28]-G, Aβ[25-42], Aβ[25-42]-G, Aβ[25-42]+G and Aβ[1-42] peptides induced a statistically significative production of IFN-γ in challenged T-lymphocytes compared to CTR and to Aβ[13-28]+G and Aβ[1-16]+G stimulation. Interestingly, these latter two peptides were able to not induce production of IFN-γ or to a very small extent respectively. Consequently, no significant cellular inflammatory activity is expected in any of the healthy donors.

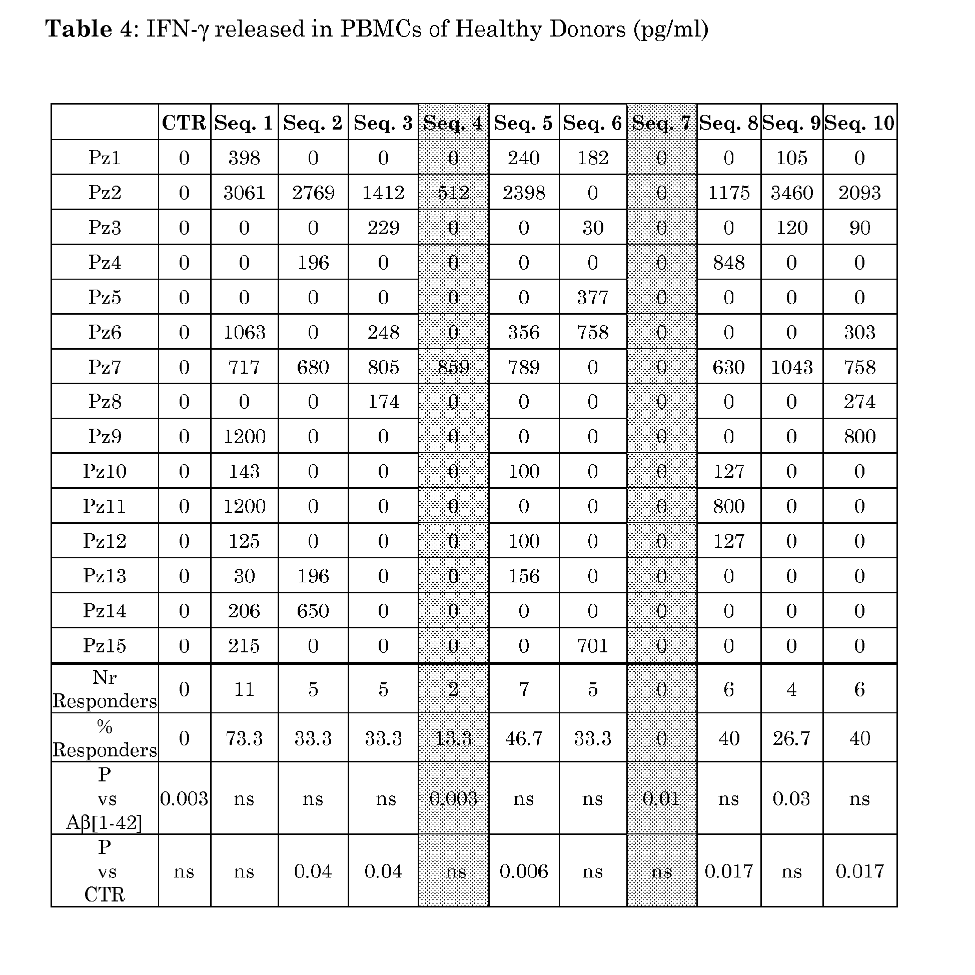

To better investigate immunological properties of selected Aβ[13-28]+G peptide, a new immunization of C57BL6 mice was performed as described by Sigurdsson and colleagues (Sigurdsson E.M., et al., Am. J. Pathol., 2001, 159, 439), with the particularity that the peptide was conjugated to KLH via glutaraldheide mediated cross-linking. Complete Freund's adjuvant was used for the first injection and incomplete Freund's adjuvant for the next injections. The mice received a subcutaneous injection of 100 µg of the peptide followed by a second injection 2 weeks later. Results confirmed that Aβ[13-28]+G peptide was able to induce specific immunogenic responses in C57BL6 mice. The peptide elicited a robust IgG response (figure 3) and a more modest IgM response (data not shown). Moreover, the produced antibodies were able to cross-react with Aβ[1-42] synthetic peptide.

## Claims

1. An antigenic peptide consisting of a fragment of the amyloid peptide Aβ within the [1-28] region ,wherein each amino acid in even position is replaced by the amino acid glycine.

2. An antigenic peptide according to claim 1 consisting of 16 or 17 amino acid residues.

3. An antigenic peptide according to any of claims 1-2 consisting of the amino acid sequence of Seq. N° 4.

4. An antigenic peptide according to any of claims 1-2 consisting of the amino acid sequence of Seq. N° 7.

5. A composition comprising at least one antigenic peptide according to any of claims 1-4, wherein the antigenic peptide is presented with a carrier selected from the group consisting of Keyhole limpet hemocyanin (KLH), ovalbumin (OVA), immunoglobulin molecules, thyroxine-binding globulin, tetanus toxoid and bovine serum albumin (BSA).

6. A vaccine comprising a composition according to claim 5 for the prevention and/or treatment of an amyloid-associated disease or condition.

7. Use of a vaccine according to claim 6 wherein the amyloid-associated disease is Alzheimer's disease.

8. Use of a vaccine according to claim 6, wherein the amyloid-associated condition is loss of memory capacity.

9. A method for stimulating the endogenous production of antibodies directed to Aβ[1-42] and Aβ[1-40] that are cross-reactive to soluble Aβ peptides and brain plaques formed therefrom by administering a composition according to claim 5 to an animal.

10. A method of producing a therapeutic vaccine comprising using a modified Aβ antigenic peptide fragment consisting of a single stretch of 16 or 17 amino acid residues according to claims 3-4, for the prevention and/or treatment of an amyloid-associated disease or condition.

11. A method according to claim 10, wherein the vaccine is obtained from the fragment [1-16] of the amyloid peptide corresponding to Seq. ID No 4.

12. A method according to claim 10, wherein the vaccine is obtained from the fragment [13-28] of the amyloid peptide corresponding to Seq. ID No 7.
